# EUROPEAN PATENT SPECIFICATION

(11) **EP 0 659 387 B1**
(45) Date of publication and mention of the grant of the patent: **16.04.2003**
(21) Application number: 94120617.9
(22) Date of filing: 23.12.1994
(51) Int. Cl.: A61B 8/12, A61N 7/00

(54) **Ultrasonic diagnosis and therapy system in which focusing point of therapeutic ultrasonic wave is locked at predetermined position within observation ultrasonic scanning range**
Vorrichtung zur Ultraschalldiagnose und -behandlung, wobei der Brennpunkt der therapeutischen Ultraschallwelle in einer vorbestimmten Lage innerhalb des Ultraschall-Beobachtungsbereiches verriegelt ist
Système de diagnostic et thérapie par ultrasons, dans lequel le point focal de l'onde ultrasonore thérapeutique est verrouillé dans une position prédéterminée dans la zone d'observation ultrasonore

(30) Priority: 24.12.1993 JP 34593493; 21.02.1994 JP 2258094; 22.02.1994 JP 2451694
(43) Date of publication of application: 28.06.1995
(73) Proprietor: Olympus Optical Co., Ltd., Tokyo 151-0072 (JP)
(72) Inventor: Fujio, Kouji, Hachioji-shi, Tokyo (JP); Hirao, Isami, Hino-shi, Tokyo (JP); Takehana, Sakae, Machida-shi, Tokyo (JP); Ueda, Yasuhiro, Tokyo (JP); Tsukaya, Takashi, Hachioji-shi, Tokyo (JP); Oaki, Yoshinao, Hino-shi, Tokyo (JP); Kuramoto, Seiji, Huntington Station, NY 11746 (US)
(74) Representative: Käck, Jürgen

(56) References cited:
- EP-A- 0 234 951
- WO-A-89/07909
- WO-A-92/15253
- US-A- 4 375 818
- US-A- 5 029 588
- INTERNATIONAL JOURNAL OF CARDIAC IMAGING , vol. 4, no. 2-4, 1989, DORDRECHT (NL), pages 79-88, XP000350877 N. BOM ET AL.: "Early and recent intraluminal ultrasound devices"

## Description

### BACKGROUND OF THE INVENTION

### Field of the Invention and Related Art and Prior Art Statement

The present invention relates to an ultrasonic diagnosis and therapy (treatment) system having an ultrasonic probe which is inserted into a body cavity or coelom to emit an observation ultrasonic wave and a therapeutic ultrasonic wave.

Generally, various kinds of ultrasonic therapy apparatuses have conventionally been proposed in which ultrasonic pulses are repeatedly sent or transmitted into organism tissues from ultrasonic transducers, echoes of the ultrasonic pulses which are reflected from the organism tissues are received by the ultrasonic transducers which are provided integrally with each other or separately from each other, and a direction in which the ultrasonic pulses are transmitted and received is gradually shifted, whereby information which is collected from a plurality of directions within the organism is displayed as visible ultrasonic tomographic or ultrasonogram images.

It is general that such ultrasonic diagnosis apparatuses are due to external ultrasonic probes.

However, probes which are combined with endoscopes, and internal ultrasonic probes such as ultrasonic probes having thin diameters, coelom ultrasonic probes inserted into the coelom, or the like, are also used widely.

Meanwhile, various kinds of ultrasonic therapy apparatuses have also been proposed such as calculus crushing apparatuses, ultrasonic thermotherapy apparatuses or the like in which various kinds of therapies are conducted by focusing the ultrasonic wave. Of these therapy apparatuses due to the ultrasonic wave, there have been ultrasonic high-temperature therapy apparatuses in which organism tissues such as cancer or carcinoma tissues or the like are instantaneously cauterized in high temperature and are treated by the focused ultrasonic wave of high strength or intensity.

As the ultrasonic high-temperature therapy apparatuses, an arrangement has been known which is provided with an internal probe building therein an ultrasonic transducer of relatively small size, and which is inserted into a rectum to treat a hypertrophied prostate, in addition to an arrangement in which ultrasonic wave is focused onto an object part from an external applicator which has an ultrasonic transducer wide in opening, in order to acquire a focused ultrasonic wave having high intensity.

For example, disclosed in PCT WO93/16641 is an arrangement in which an ultrasonic transducer which is capable of conducting linear and sector scanning is arranged at a distal end of a probe, and an ultrasonic wave having weak energy is scanned to acquire an observation image, to thereby detect a position of an object part to be treated and, thereafter, the position of the ultrasonic transducer is so set as to be capable of irradiating the ultrasonic wave to the object part to be treated, and the ultrasonic wave having strong or high energy is irradiated to conduct therapy.

This first prior-art example has the possibility that function thereof is dropped or reduced less than case where exclusive ultrasonic transducers are provided, because the ultrasonic transducers are used for observation and for therapy, respectively.

For example, in case where observation is conducted the energy strength may be weak and, accordingly, the size of the ultrasonic transducer may be small or low.

Meanwhile, in case where therapy is conducted, a high ultrasonic wave becomes necessary or is required and, accordingly, it is better that the size of the ultrasonic transducer is large as far as possible. Further, it is necessary to scan the ultrasonic wave in order to conduct the observation.

Accordingly, in order to cause the ultrasonic transducer to have both functions like the prior art example, it will be required to or be necessary to make the relatively large ultrasonic transducer to an arrangement capable of being rotated. or the like. Moreover, in order to position an irradiation part of the ultrasonic transducer so as to be capable of irradiating the therapeutic ultrasonic wave toward the object part to be treated, it is required that scanning is first made by the ultrasonic wave having weak or low energy and, thereafter, an irradiation direction is locked to the object part.

There is the possibility that. if there is relative movement between the probe and the coelop wall or the like with which the probe is in acoustic contact, or the like within the time until the locking is conducted, the therapeutic ultrasonic wave is irradiated to parts which are different from the object part.

Specifically, in the prior-art example, operation or work is required or necessary that the ultrasonic wave is scanned under the observation state, a position of the object part is detected from an observation image thereof, the ultrasonic transducer is set to a scanning position where the ultrasonic wave can be outputted with respect to the aforesaid position, and setting is made to a stage capable of outputting the therapeutic ultrasonic wave so as to be focused. In addition, since the scanning position is locked under the set state. the state becomes a state where the observation image cannot be acquired. For this reason, even if there is a change in state after having been set, it is impossible to confirm the change in state.

It is desirable in order to secure the safety as the therapy apparatus that, in case where the therapeutic ultrasonic wave is outputted, it is possible to confirm, from the ultrasonic observation image, or the like, that a focusing point of the therapeutic ultrasonic wave is set to the object part to be treated, or it is desirable to have equivalent function.

In the prior-art example, it is necessary to scan the ultrasonic wave in order to conduct the confirmation. The lock must be released in order for the scanning.

Further, in order to confirm the treated or cured state of the object part in case where the therapeutic ultrasonic wave is irradiated to conduct the high-temperature therapy, it becomes necessary to scan the ultrasonic wave to acquire the image.

In case where the therapeutic ultrasonic wave is irradiated to conduct the high-temperature therapy, it is necessary that an amount of irradiation is an adequate amount. If excessive irradiation is conducted, peripheral parts will also be treated in high temperature. For this reason, it is desirably to confirm, by the ultrasonic observation image, whether or not, after the object part has been irradiated by an adequate amount of irradiation, the object part is treated in high temperature by the amount of irradiation.

In the prior-art example, also in case where the confirmation is conducted, it is necessary to change over to an observation state to conduct the scanning of the ultrasonic wave. Moreover, in case where irradiation is necessary, re-setting must be conducted to a positioning position.

Furthermore, there are many cases where, in the prior-art example, since the probe is rigid, it becomes difficult that the irradiation part which irradiates the ultrasonic wave, on the distal side of the probe is focused to the object part so as to be made to a state capable of irradiating. That is, since the ultrasonic wave is outputted to the side of the probe, in order to set the object part at least to the side of the probe, the insertion direction of the probe is restrained to a narrow range or scope. On the contrary, if the construction is a construction which has a curvature part at the insertion part, this restriction can remarkably be solved.

Meanwhile, Japanese patent Unexamined Publication No. SHO 62-127050 (127050/1987) discloses an ultrasonic endoscope which is provided with an ultrasonic probe of structure in which an observation ultrasonic transducer and a therapeutic ultrasonic transducer are arranged at a distal part of an endoscope insertion part such that their back-face sides are joined to each other, and both the ultrasonic transducers are mounted on a distal end of a shaft which is rotated.

The second prior-art example has the observation transducer and the therapeutic transducer. However, since the second prior-art example is of structure which is rotated about a common shaft, the therapeutic transducer is also rotated in case where the observation is conducted. Further, in order to conduct the therapy, it is necessary to stop scanning of the observation transducer. Accordingly. the second prior-art example has probiems similar to those of the first prior-art example, except for a merit having the curvature part.

In a first embodiment of WO-A-92/15 253 a device is disclosed provided with a therapeutic ultrasonic transducer and an observation ultrasonic transducer for observation purposes. The therapeutic ultrasonic transducer generates a focussing US beam perpendicular to the probe which is directed to a tissue (e.g. cancer) to be treaded. The observation ultrasonic transducer has an oblique field of view which intersects the treatment US beam direction including the focus point thereof. In a further embodiment, an optical fibre terminating near the therapeutic ultrasonic transducer is provided instead of the observation ultrasonic transducer. Again the field of optical observation is inclined so that it intersects the beam path of the therapeutic beam.

In WO-A-89/07 909 a device is disclosed wherein a visualization element which includes in its interior space an ultrasonic transducer for observation is inserted in a probe having therein an ultrasonic transducer for therapy which is inserted into the rectal area. In such a device it is possible that the driving mechanism to rotate the ultrasonic transducer provided in the probe impedes the insertion of the visualization element into the probe.

It is an object of the invention to provide an ultrasonic probe and a system including such a probe which efficiently provide different observation principles in a compact insertion part.

This object is solved by the ultrasonic probe of claim 1 and the ultrasonic diagnosis and therapy system of claim 8.

Particular embodiments of the invention are set out in the depending claims.

Fig. 1 to Fig. 8 relate to an embodiment of the invention. Fig. 1 being an arrangement view showing an arrangement of an ultrasonic diagnosis and therapy system according to the seventh embodiment:

Fig. 2 is a perspective view showing an outer appearance of an ultrasonic probe in the seventh embodiment;

Fig. 3 is a perspective view showing an outer appearance of an ultrasonic probe according to a modification of Fig. 2;

Fig. 4 is a perspective view showing a distal-end side of the ultrasonic probe;

Fig. 5A is a view showing a distal-end side of an ultrasonic probe according to a first modification;

Fig. 5B is a top plan view of Fig. 5A;

Fig. 5C to Fig. 5E are views showing shapes or forms of a therapeutic ultrasonic transducer;

Fig. 6 is a perspective view showing a distal-end side of an ultrasonic probe according to a second modification;

Fig. 7 is a perspective view showing a distal-end side of an ultrasonic probe according to a third modification;

Fig. 8 is an explanatory view showing a use example, of operation description of the embodiment.

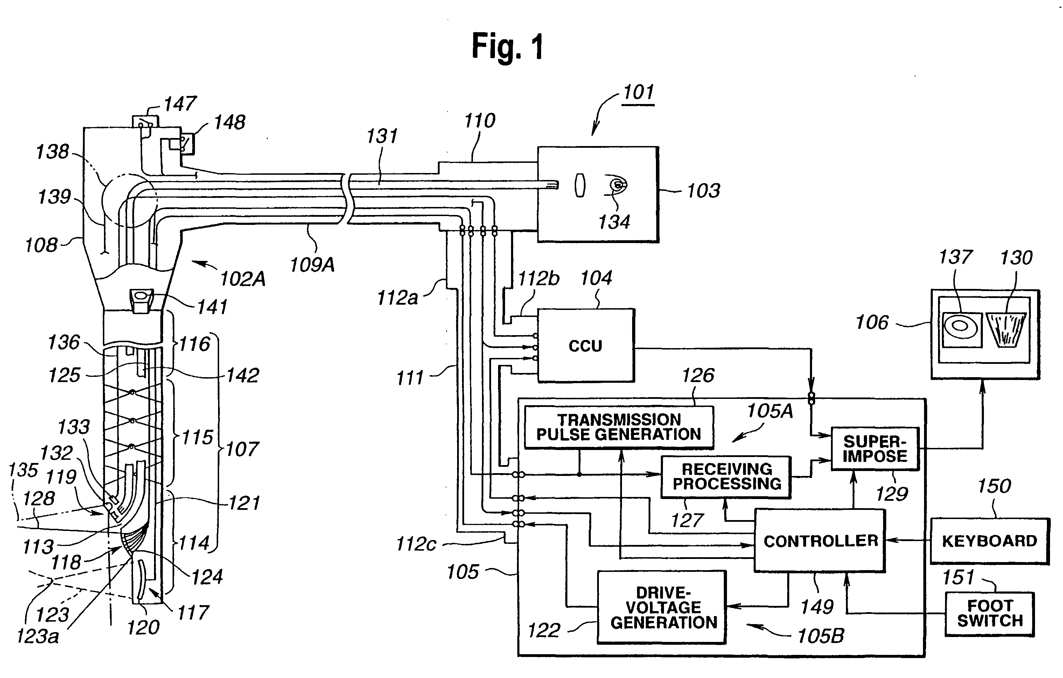

As shown in Fig.1, the embodiment of the ultrasonic diagnosis and treatment system 101, for example, comprises a ultrasonic probe 102A having an electronic-endoscope-like structure, a light source device 103 for providing illumination to light transmission means of the ultrasonic probe 102A, a camera control unit (referred to as CCU, hereinafter) 104 for signal processing for image pickup means built in the ultrasonic probe 102A, an ultrasonic diagnosis and treatment apparatus 105 housing a signal processing system 105A for producing ultrasonogram and a treatment signal generator system 105B for producing treatment driving signal, and a color monitor 106 for presenting both an endoscopic image and an ultrasonogram based on the video signal from CCU 104 and the video signal derived from the ultrasonic signal processing system.

The ultrasonic probe 102A is constructed of a small tube (capillary) insert portion 107, a wide operation portion 108 which is formed on the proximal of the insert portion 107 and which is gripped by an operator for insertion and bending operations, and a universal cable 109A extending from the operation portion 108, wherein the light source connector 110 at one end of the universal cable 109A is detachably connected to the light source device 103.

Connected to the light source connector 110 is a signal connector 112a at one end of a signal cable 111. The other ends of the signal cable 111 have a CCU connector 112b to be connected to the CCU 104 and an ultrasonic connector 112c to be connected to the ultrasonic diagnosis and treatment apparatus 105.

The insert portion 107 is constructed of a rigid distal-end portion (or end portion) 114, a bendable portion 115, and a flexible portion along the axis of the insert portion 107 way back from its end. As shown in Fig. 27, the end portion 114 is constructed of ultrasonic treatment means 117, ultrasonic monitoring means 118, an forceps outlet port 113 as a treatment function guide portion, and optical monitoring means 119 along the axis of the insert portion 107 way back from its end.

The ultrasonic treatment means 117 is made of a ultrasonic treatment transducer 120 having a concave face. By applying a driving signal to the transducer 120 from a driving voltage generator 122 in the ultrasonic diagnosis and treatment apparatus 105, via a signal line 121 that is routed through the insert portion 107, the ultrasonic wave emitted from the concave face of the therapeutic ultrasonic transducer travels keeping its wave front concave, converges in a beam coverage 123 shown by the dotted line in Fig.1 and still travels as a directional therapeutic ultrasonic beam that focuses at a focus 123a.

The ultrasonic beam is very intensive at the focus 123a, and thus gives a high acoustic energy density. When with a lesion such as tumor set in the vicinity of the focus 123a, a large amplitude driving signal is applied to the concave therapeutic ultrasonic transducer 120, a high acoustic energy derived from generated ultrasonic wave burns the lesion for cauterization treatment.

The ultrasonic monitoring means 118 is made of a convex-array ultrasonic transducer 124. which is connected via a signal line 125 to a transmission pulse generator circuit 126 and a receiver signal processing circuit 127, both of which form a signal processing system 105A.

A transmission pulse from the transmission pulse generator circuit 126 is sequentially applied to each transducer element of the convex-array ultrasonic transducer 124, which scans sequentially a radially extending ultrasonic monitoring coverage 128 in a plane in which the axis of the insert portion 107 lies, in order to irradiate a target lesion. The ultrasonic wave reflected back from the lesion is then received by each transducer element of the convex-array ultrasonic transducer 124 that is already used for transmission, and there converted into an electrical signal. The electrical signal is subjected to the receiver signal processing circuit 127 for signal processing, including amplifying, then converted via DSC into a standard video signal corresponding to ultrasonogram, and fed to a color monitor 106 via a superimposing circuit 129 so that ultrasonogram 130 as an ultrasonic image is presented thereon.

The driving frequency of the transmission pulse from the transmission pulse generator circuit 126 is set such that it does not agree with the frequency (or frequency band) of the driving signal from the driving voltage generator circuit 122.

Ultrasonogram image is not interfered with by any noise due to treatment ultrasonic wave even if the treatment ultrasonic wave is emitted while ultrasonogram monitoring is made using the ultrasonic monitoring means 118. In this case, a filter such as a trap circuit for filtering out noise may be included in the receiver signal processing circuit 127. A filter such as a trap circuit may be included to the driving voltage generator circuit 122 to filter out the driving frequency component from the ultrasonic monitoring means 118.

As shown in Fig.1 the optical monitoring means 120 is constructed of illumination light projection means (or simply illumination light system) for projecting illumination light through a light guide 131, a lens and an illumination window, and image pickup means having an objective lens 132 for picking up an image of the tissue of interest lighted by the illumination, and CCD 133 disposed in a plane of the focus of the objective lens 132.

The light guide 131 is routed through the insert portion 107, and its light source connector 110 is connected to the light source 103. The light guide 131 transmits illumination light, generated by a lamp 134 and converged by the lens, and projects in a slantly forward direction the illumination light through the end face of the end portion 114 and the lens of the illumination window.

The coverage of the illumination light looking in a slantly forward direction almost agrees with the viewing coverage 135 of the objective lens 132.

The optical monitoring means 120 is thus constructed of slantly forward looking illumination and monitoring means. The objective lens 132 allows an optical image of the tissue of interest to take place on the pickup surface of CCD 133. The CCD 133 is connected to an unshown video signal processing system in CCU 104 via a signal line 136. The signal from CCD 133 is converted by the video signal processing system into a video signal. which is then fed via the superimposing circuit 129 to the color monitor 6 where both ultrasonogram 130 and endoscopic image 137 are simultaneously displayed.

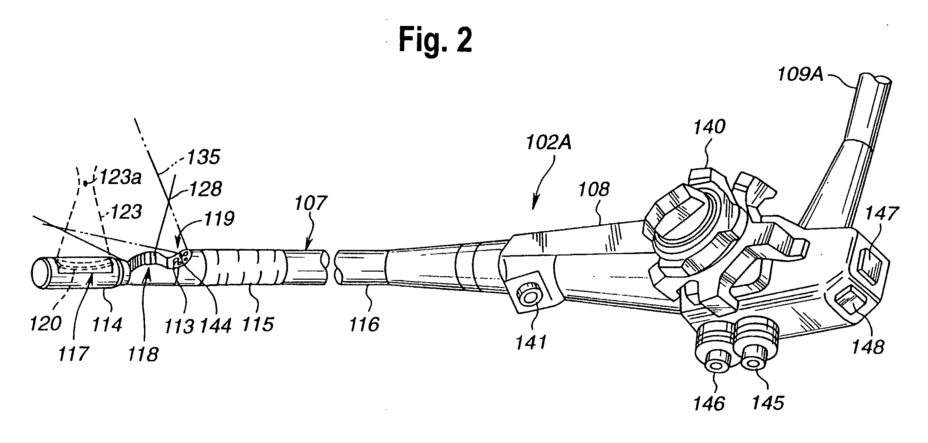

The bendable portion 115 connected to the end portion 114 is constructed of a number of segments which are mutually connected in a flexible manner to allow a bending. Angle wires 129 are connected at their one ends to the end portion 114, and connected at the other ends to a pulley 38 in the operation portion 108. Rotation of an angle knob 140 (Fig.2) turns the pulley 138 which in turn pulls one of the angle wires 129 and loosens the other of the angle wires 129 so that a bend is formed in a manner that the bendable portion 115 is bent inwardly on the pulled wire 129 side.

The manipulation of the angle knob 140 allows bending in four directions. up and down. right and left, or two directions up and down. or right and left.

The bendable portion 115 is freely bent. causing the ultrasonic treatment means 117, the ultrasonic monitoring means 118, the treatment function guide portion, and the optical monitoring means 119 all in front of the bendable portion 115 to look in any direction, and the construction of the bendable portion 115 has a construction similar to the bendable portion of the ordinary endoscope.

The operation portion 108 has on its distal portion an inlet port 141 through which an instrument such as forceps is introduced. The inlet port 141 communicates with an instrument channel 142 disposed in the insert portion 107.

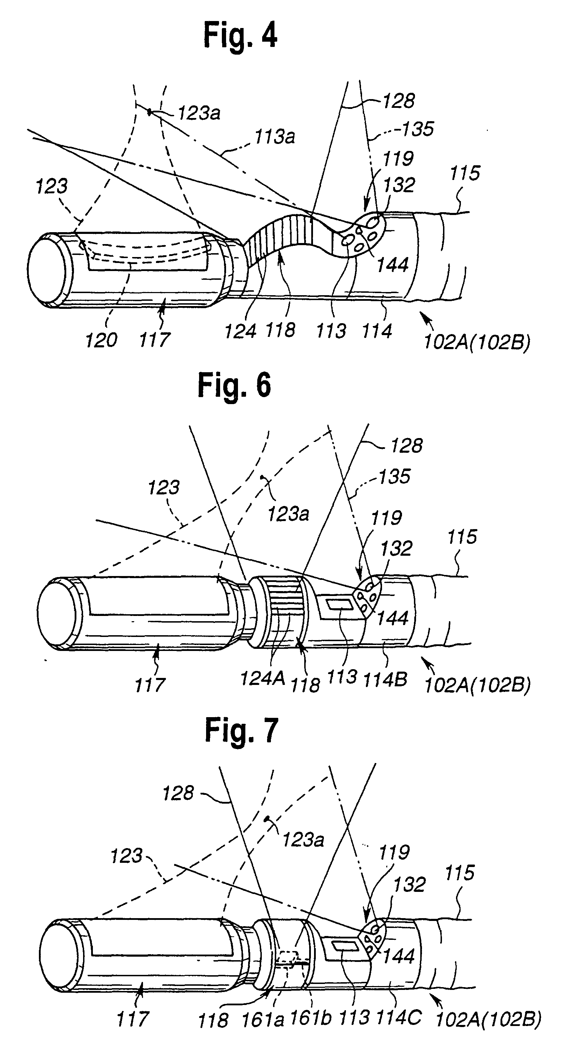

As shown in Fig.4, the instrument channel 142 communicates with the treatment function guide disposed in the end portion 114, that is, the forceps outlet port 113 which functions as a guide for treatment using an instrument.

The forceps outlet port 113 is disposed in the vicinity of the optical monitoring means 119. When an unshown instrument is projected out of the forceps outlet port 113 for treatment of an organ, the optical monitoring means 119 closely observes the projected instrument and the state of the treatment.

As shown in Fig.4, a nozzle 144 is disposed facing an observation window in order to clean and dry the outer surface of the observation window. The nozzle 144 communicates with air and water pipings (not shown) routed through the insert portion 107, and their other ends are connected to a fluid control system in the light source device 103. By manipulating air feeding and water feeding buttons 145 (see Fig.2) on the operation portion 108. air feeding or water feeding is activated. When the outer surface of the observation window is stained, it is cleaned by feeding water.

A subsequent air feeding dries or eliminates water drops from the outer surface of the observation window. Thus, the observation window is kept clean to maintain clear viewing therethrough.

A suction button 146 is provided next to the air and water buttons 145, and the suction button 146 is manipulated to suck up unwanted humor via the forceps outlet port 113 on the end of instrument channel 142, and to drain the unwanted humor into a disposal container. On its proximal portion, the instrument channel 142 communicates with an unshown suction channel which is connected to a suction pump in the fluid control system. The instrument channel 142 partly functions as a suction channel for sucking up a fluid, and in this case. a suction port is the forceps outlet port 113.

Disposed on the proximal portion of the operation portion 108 are video control buttons (or video control switches) 147, 148 for freezing the endoscopic image or switching the display, and the signals from video control buttons 147, 148 are sent to CCU 104 and a controller 149.

The controller 149 is connected to a keyboard 150 and a foot switch 151. Through the keyboard 150. treatment time may be set for the ultrasonic treatment means 117 or clinical recording of a patient may be input. By manipulating the foot switch 151, the ultrasonic treatment means 117 is activated for treatment and then deactivated.

The controller 149 is also connected to the driving voltage generator 122, the transmission pulse generator circuit 126, the receiver processing circuit 127, and the superimposing circuit 129 all in the ultrasonic diagnosis and treatment apparatus 105. By entering inputs through the keyboard 150, the controller 149 sets parameter settings such as an intensity (or amplitude) of the driving voltage to the driving voltage generator 122 for the ultrasonic treatment means 117 to provide high-power ultrasonic output, modifies ultrasonic transmission settings for ultrasonogram, modifies signal processing settings for received signal, and controls images displayed on the color monitor 106.

The controller 149 is also connected to CCU 104, and thus the keyboard 150 controls CCU 104, for example, to set CCU4 to a mode for a still picture presentation. In Fig. 1 the ultrasonic diagnosis and treatment apparatus 105 comprises the signal processing system 105A for providing ultrasonogram and the treatment signal generator system 105B for producing the treatment signal. Alternatively. however, the signal processing system 105A and the treatment signal generator system 105B may be separately housed. Furthermore, two monitors may be included in the system, one for the endoscopic image presentation and the other for the ultrasonogram presentation.

Fig.2 shows the outline of the ultrasonic probe 102A. The ultrasonic probe 102A employs the configuration of an electronic endoscope in which image pickup means is used as the optical monitoring means 119. Although in the ultrasonic probe in Fig.1, the configuration of the electronic endoscope is used as the optical monitoring means 119, alternatively, an ultrasonic probe 102B having the configuration of a fiberscope may be used as the optical monitoring means 119.

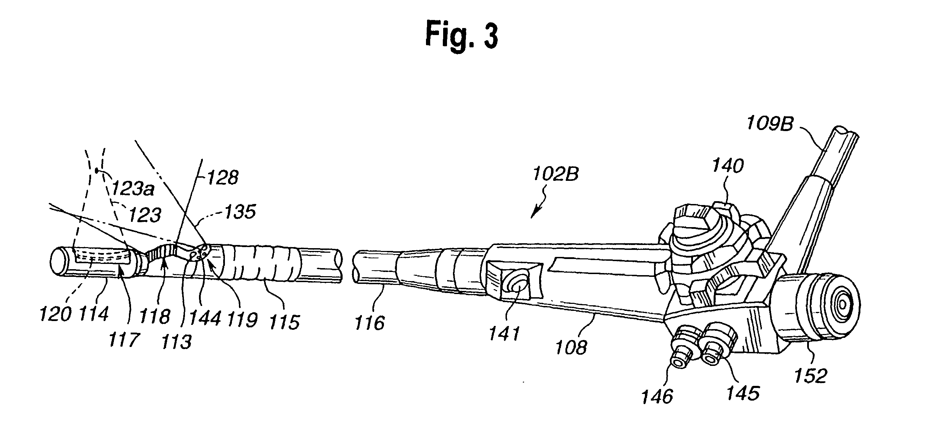

As shown in Fig.3 the ultrasonic probe 102B is dispensed with the video control buttons 147. 148 on the proximal end of the operation portion 108 in the ultrasonic probe 102A in Fig.25, and instead, the ultrasonic probe 102B is provided with an eye-piece portion 152, instead of CCD 133, an image guide that transmits an optical image is disposed, and the image guide transmits the optical image obtained by the objective lens 132 to the end face of the eye-piece portion 152, and an enlarged optical image is observed through the viewing window on the proximal end of the eye-piece portion 152.

A universal cable 109B having no signal line 136 is extended from the operation portion 108. The rest of the construction of the ultrasonic probe remains unchanged from Fig.2. Reference numerals equivalent to those described with reference to Fig.2 are designated with the same reference numerals. and their description is omitted. As an ultrasonic probe, an unshown TV camera is mounted on the eye-piece portion 152 of the ultrasonic probe 102B. If, in this case, the signal connector at the signal cable of the TV camera is connected CCU 104 in Fig.1 an ultrasonic diagnosis and treatment system having almost the same functions described with respect to Fig.1 is provided.

The alternate embodiment of the ultrasonic diagnosis and treatment system constructed of the ultrasonic probe 102B in Fig.3 is equivalent to the system without CCU 104 in Fig.1. In this case, the color monitor 106 presents the ultrasonogram 130 only (namely, the superimposing circuit 129 outputs its video input from the receiver processing circuit 27 as it is without any processing, or the superimposing circuit 129 may be dispensed with).

Fig.4 is an enlarged view showing the ultrasonic probe 102A or 102B shown in Fig.2 or Fig. 3.

As shown in Fig.4, the ultrasonic treatment means 117 is constructed of a concave ultrasonic treatment transducer 120 having a rectangular transducer element which is inwardly curved along its longitudinal direction. Coverage overlapping takes place by three coverages: the beam coverage 123 of the treatment ultrasonic beam emitted by the ultrasonic treatment transducer 120 in the transverse direction (upward direction in Fig.4) approximately perpendicular to the transducer longitudinal direction. the sectorial ultrasonic monitoring coverage (simply monitoring coverage) 128 looking slantly forwardly. resulted in by, for example. the convex-array ultrasonic transducer 124 as the ultrasonic monitoring means 118, and the conical monitoring coverage 135 by the optical monitoring means 119.

More specifically, as shown in Fig.4, the optical monitoring means 119 is set up so that the focus 123a is kept within its monitoring coverage 135, and the ultrasonic monitoring means 118 is also set up so that the focus 123a is kept within its ultrasonic monitoring coverage 128. For example, the ultrasonic monitoring coverage 128 is sectorially wide between an angle transverse to the probe axis and an angle slanted forwardly in Fig. 4, and the sector center of the ultrasonic monitoring coverage 128 is slanted forwardly.

Since the optical monitoring coverage and the acoustic monitoring coverage overlap with the focus 123a kept within them, with a lesion set in the vicinity of the focus 123a, the ultrasonogram 130 and the endoscopic image 137 show the state with the lesion in presence (In the endoscopic image 137, however, a lesion cannot be visibly recognized if the lesion exists under a top surface. In this case, the lesion can be recognized in the ultrasonogram 130.)

The above arrangement offers the advantage that positioning the end of an instrument exactly on a lesion is easily performed, and this advantage is particularly useful in such a situation that a lesion needs to be partly removed for biopsy using an instrument before cauterizing the lesion with the ultrasonic treatment means 117 and that biopsy is performed to assure that a lesion has been cauterized sufficiently enough after the lesion has been treated by the ultrasonic treatment means 117.

The ultrasonic treatment transducer 120 constituting the ultrasonic treatment means 117 is used to emit a high-powered, converged beam (namely, focussed ultrasonic beam) for treatment, and may be a single ultrasonic transducer or a plurality of ultrasonic transducers.

The ultrasonic transducer 120 is not limited to a longitudinally concave one, the ultrasonic transducer 120 may be constructed of a single or a plurality of longitudinally concave transducers or transversely concave transducers. If planar transducers are employed, they may be constructed in a phase-array arrangement so that phase is shifted to focus the ultrasonic beam.

A concave lens may be attached to the ultrasonic output face of a planar ultrasonic transducer so that the beam is emitted to be focussed. The construction of the transducer has no particular limitation in its design as long as the transducer has a construction suited for emission to focus the ultrasonic beam and an associated driving means.

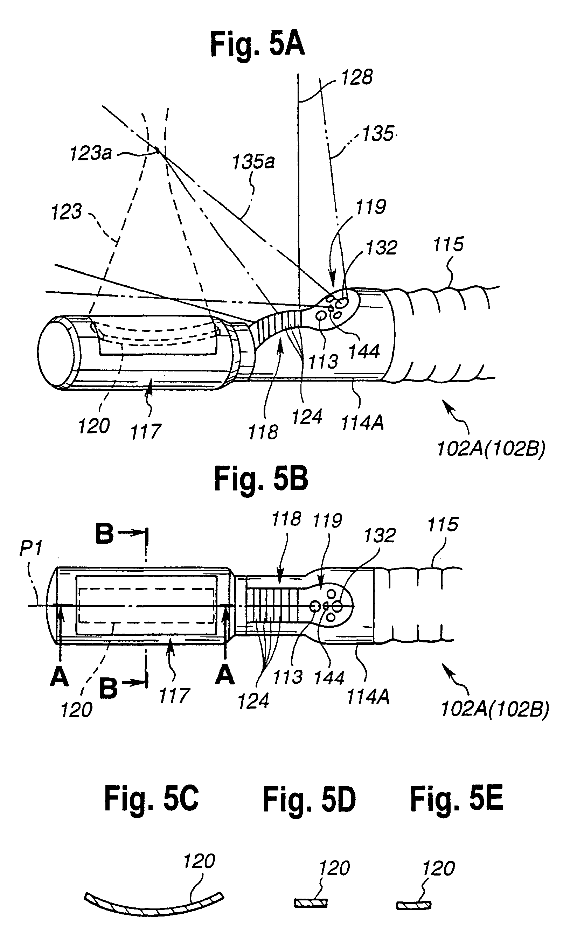

The construction of the end portion 114 is not limited to the one show in Fig.4, it may be constructed as shown in Fig.5A through Fig.7.

In Fig.4, the ultrasonic monitoring means 118 provides the sectorial ultrasonic monitoring coverage 128 ranges from an angle transversely extending to an angle slanted forward. In a first alternate example of the end portion 114A shown in Fig.28A, the center axis of the monitoring coverage 135. namely, monitoring center axis (or optical axis) of the optical monitoring means 119 is designed to intersect the focus 123a, and the center axis 128a of the ultrasonic monitoring coverage 128 of the ultrasonic monitoring means 118 is designed to intersect the focus 123a.

As shown in Fig.5B, the concave faced ultrasonic transducer 120, the convex-array ultrasonic transducer 124, and the objective lens 132 are arranged on the end portion 114 so that the center cross-section line P1 that longitudinally extends along the ultrasonic treatment transducer 120 made of a concave faced rectangular ultrasonic transducer board runs through the center of the convex-array ultrasonic transducer 124 and further runs through the center line (optical axis).

In Fig.5B, the illumination windows are symmetrically arranged on both sides of the objective lens 132 (in Fig.4, the two illumination windows are arranged on one side of the objective lens 132, but even in Fig. 4, the two illumination windows may be arranged on both sides of the objective lens 132 as in Fig.25). In Fig.5A, the forceps outlet port 113 and the nozzle 144 are aligned with the center cross-sectional line. Each of the illumination windows is provided with the illumination lens shown in Fig.1 under an unshown glass cover which functions as a protective member.

The observation window is also closed by an unshown glass cover which functions as a protective member, and the objective lens 132 is disposed in the inner region protected by the glass cover. As shown in Fig.1, the objective lens 132 may be directly exposed on the observation window.

As seen from the arrangement in Fig.5B, the ultrasonic monitoring coverage 128 of the convex-array ultrasonic transducer 124 lies in the cross-section P1, and is a sector looking slantly forward. The optical monitoring coverage 135 of the optical monitoring means 119 is a cone centered on the monitoring center axis 135a.

In Fig.5B, the beam coverage 123 of the treatment ultrasonic beam, the ultrasonic monitoring coverage 128, and the monitoring coverage 135 overlap. As already described, the center axis 128a of the ultrasonic monitoring coverage 128 is designed to intersect the focus 123a, and the center axis 135a of the monitoring coverage 135 of the optical monitoring means 119 is designed to intersect the focus 123a, and position setting (or positioning) is thus easily performed in treatment.

The concave faced ultrasonic transducer 120 has an arc like concave shape along its longitudinal direction as shown in Fig.5C which is the view taken along A-A line in the plan view in Fig.5B. Electrodes are installed on both surfaces of the concave transducer, and by applying a driving signal to them. ultrasonic wave is emitted upward. A damping material is attached to the back surface of the concave surface.

The transducer may be planar as shown in Fig.5D which is taken along B-B line in Fig.5B or may be transversely concave as shown in Fig.5E. The radius of curvature in the concave in Fig.5C is designed to be equal to that in the concave in Fig.5E, and the emitted ultrasonic wave is focused at the same point.

When the width of the transducer is small, it may be planar as shown in Fig.5D, and when the width of the transducer is large, it may be curved as shown in Fig.5E to transversely focus the beam. The rest of the construction remains unchanged from the construction in Fig.4. The first alternate example has almost the same functions as the end portion 114 in Fig.4. In addition to the advantages of the seventh embodiment, this alternate example offers an additional advantage in which positioning is more easily performed because the center axis 128a of the ultrasonic monitoring coverage 128 is designed to intersect the focus 123a.

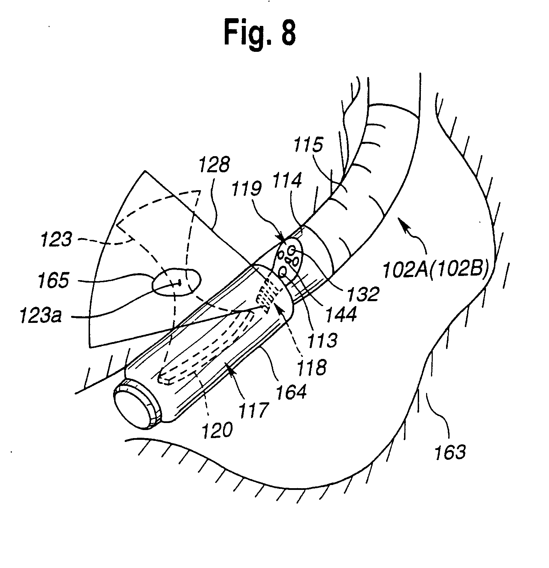

In a second alternate example with the end portion 114B in Fig.6. the ultrasonic monitoring means 118 is made of an electronic radial-array ultrasonic transducer 124A, rather than the convex-array transducer 124 in Fig.4 or Fig.5A. Specilically, a number of rectangular transducers are arranged on the cylindrical surface of the end portion 114B (for example, over a certain sector if viewed from the center line of the cylinder). By allowing each transducer to transmit and then receive, the ultrasonic monitoring coverage 128 is a sector with respect to a plane perpendicular to the axis of the end portion 114B.

Although in Figs.4 and 5A, the forceps outlet port 113 is circular, it is now square in Fig.6. Also in Fig.6, the ultrasonic treatment means 117 is disposed so that the treatment ultrasonic beam 123 is slanted backward, and the focus 123a of the treatment ultrasonic beam 123 is designed to come to the center of a plane in which the ultrasonic monitoring coverage 128 lies. The advantage of this alternate example is similar to that of the first alternate example.

In the end portion 114C in Fig.7, the ultrasonic monitoring means 118 is made of a mechanical radial scanning ultrasonic transducer 161a rather than the electronic radial array ultrasonic transducer 124A in Fig.4. The mechanical radial scanning ultrasonic transducer 161a has a planar ultrasonic transducer 161a on the distal end of a flexible shaft 161b, and the proximal end of the flexible shaft 161b is rotated or reciprocated by an unshown motor. Thus, the ultrasonic transducer 161a is rotated together, or reciprocated, and as a result, ultrasonic wave is emitted in a sector with respect to a plane perpendicular to the axis of the insert portion 107.

The ultrasonic monitoring means 118 is not limited to Figs.4 through 7, and any other construction will do if it provides ultrasonogram.

The optical monitoring means 119 exemplifies a slantly looking optical system as preferred in the embodiment and alternate examples, but the optical monitoring means 119 is not limited to this. Other optical systems having a different viewing field are equally acceptable.

The operation of the embodiment is now discussed referring to Fig.8.

As shown in Fig.8, the ultrasonic probe 102A (102B) constructed as above is inserted into a tract organ 163 such as the stomach. The end portion 114 is put into contact with a region of the tract wall where diagnosis and treatment are required. referring to the optical image from the optical monitoring means 119 and the ultrasonogram from the ultrasonic monitoring means 118 while performing bending operation.

As shown in Fig.8. the ultrasonic treatment means 117 and the ultrasonic monitoring means 118 at the end portion are covered with a balloon 64 which is filled with ultrasonic transmittable liquid. The treatment ultrasonic wave and the monitoring ultrasonic wave are transmitted and received via the ultrasonic transmittable liquid within the balloon 164.

When a clear vision is obstructed because the optical monitoring means 119. specifically, the outer surface of the observation window that houses the objective lens 132 is stained with mucus or humor in the course of insertion of the ultrasonic probe 102A (102B) or contacting the end portion 114, water or air is fed through the nozzle 144 to clean or dry the observation window. After a clear vision is assured, insertion operation is assumed.

The forceps outlet port 113 as the treatment function guide and its channel 142 are used to suck up humor or mucus. By operating the suction button 146 on the operation portion 108, mucus or humor is sucked up by an external suction pump. Each function of the ultrasonic probe 102A (102B) can be used in the same way an ordinary endoscope or an ultrasonic endoscope is inserted.

By allowing an unshown instrument to project from the forceps outlet port 113, for example, allowing a biopsy needle to pierce a lesion, a tissue may be sampled for examination and diagnosis.

When a lesion 165 is acquired within the ultrasonic monitoring coverage 128, and imaging is completed, the focus 123a of the treatment ultrasonic beam given by the ultrasonic treatment means 117 is aligned to the lesion 165. This is performed referring to a marker given in the ultrasonogram and the image of the lesion. The position of the end portion 114 of the ultrasonic probe 102A (102B) is set by bending operation of the bendable portion 115. In this manner, the focus is positioned such that the focus 123a is placed at the lesion 165.

After positioning the focus, the foot switch 151 is pressed to allow the driving voltage generator 122 to apply a large driving voltage, and the ultrasonic treatment means 117 outputs treatment ultrasonic wave at a power level required for cauterization. This high-powered treatment ultrasonic wave instantly raises temperature at the lesion 165, and cauterizes the lesion.

Observing denatured protein as a result of cauterization through ultrasonogram from the ultrasonic monitoring means 118, high-powered treatment ultrasonic beam is directed at a lesion until denature of protein is sufficient enough to necrotize the lesion.

Then, irradiation with high-powered ultrasonic beam is stopped, treatment rate is confirmed referring the ultrasonogram. If the treatment is not sufficient enough, ultrasonic irradiation is continued further, and if a different part needs ultrasonic irradiation, it is also irradiated until full treatment is complete.

According to the embodiment, the ultrasonic treatment means 117, the ultrasonic monitoring means 118. the treatment function guide, and the optical monitoring means 119 are configured on the distal end of the bendable portion 115 in an advantageous manner that permits optical monitoring, ultrasonic diagnosis and ultrasonic treatment.

The end portion 114 is inserted into the superior digestive tract or the inferior digestive tract observing an optical image, and ultrasonic thermotherapy (cauterization) can be applied to the wall surface of the digestive tract or inner organs beneath the wall surface observing ultrasonogram.

Thus, the use of the ultrasonic diagnosis and treatment system 1 greatly expands the application range of ultrasonic diagnosis and treatment.

The conventional ultrasonic probe with no bendable portion limits the sectorial range in which the ultrasonic treatment means is put into contact with the organ in which a lesion exists. The conventional probe is inserted almost in a linear manner and its end portion touches, on its side, an organ. The conventional probe is effectively used only when the lesion exists in the organ into its deep region that corresponds to the organ surface touched by the probe. Even in this case, an insertion opening is required, and if for example, making an insertion opening to the heart is an extremely risky task. Furthermore, costa presents difficulty inserting the probe. Thus, the range of application, in practice, is even narrower.

In contrast, since the first embodiment is provided with the bendable portion 115, bending the bendable portion 115 allows the side of the end portion 114 to be in contact with the organ in which a lesion exists.

Since the insert portion 107 of the first embodiment is flexible. the probe can be inserted into the body cavity through the oral cavity, the probe is inserted into organs in the body cavity without insertion opening to the abdomen and the like. In this case, insertion and positioning to a target organ is aided by observing the optical monitoring means 119. As in the endoscope, the end portion 114 can be set in the vicinity of the organ of interest, under observation using the optical monitoring means 119, and then the side of the end portion 114 is put into contact with the organ so that setup is completed to obtain ultrasonogram or echo of the lesion. When the focus 123a is aligned with the lesion, cauterization treatment is performed with the ultrasonic treatment means 117.

The embodiment is thus used in a wider region than the ultrasonic probe with no bendable portion. Furthermore, since a predetermined point of the ultrasonic monitoring coverage 128 of the ultrasonic monitoring means 118 comes to the focus 123a of the ultrasonic beam derived from the ultrasonic treatment means 117, positioning for cauterization in ultrasonogram is easy and position determined can be confirmed. A lesion to be cauterized may be accurately set in the vicinity of the focus 23, and reliable cauterization results. Cauterizing normal part erroneously can be avoided.

In positioning operation as above, the ultrasonic monitoring means 118 and the ultrasonic treatment means 117 are provided on the hard end portion 114. If the bendable portion 115 on the proximal side is arbitrarily bent, the position of the focus 123a practically remains anchored to a predetermined position of the ultrasonic monitoring coverage 128 of the ultrasonic monitoring means 118. Therefore, the lesion to be cauterized can be accurately set to the focus 123a, and a reliable cauterization treatment is achieved.

Since the forceps outlet port 113 is disposed on the end portion 114, sampling the tissue of a lesion assures reliable diagnosis and treatment. In this case, if the guiding direction of the forceps outlet port 113 is set to point to the focus 123a, sampling operation is easy.

In the embodiment and its alternate examples, the treatment function guide may be dispensed with, or it may be considered as part of the function of the optical monitoring means 119. In this case. the end portion 114 is constructed by arranging. from the distal side, the ultrasonic treatment means 117, the ultrasonic monitoring means 118. and the optical monitoring means 119. The bendable portion 115 is disposed on the proximal side of the end portion 114.

In the embodiment and its alternate examples, the ultrasonic probe 102A or 102B comprises. on the end portion 114 from its distal end, the ultrasonic treatment means 117, the ultrasonic monitoring means 118, the treatment function guide portion, and the optical monitoring means 119, and the bendable portion 115 on the proximal side of the end portion 114.

## Claims

1. An ultrasonic probe (102A, 102B ) comprising:
an elongated insertion part (107) insertable into coelom;
a focused therapeutic ultrasonic transducer ( 120) placed along the distal end of said insertion part, for emitting a therapeutic ultrasonic wave for conducting therapy on a subject;
an observation ultrasonic transducer (118 ) for acquiring an ultrasonic image by transmitting and receiving an observation ultrasonic wave over a scanning range which includes a direction in which said therapeutic ultrasonic wave is emitted, the observation ultrasonic transducer (118 ) being placed along the distal end of said insertion part (107);
lock means for locking a focussing point (123a) of said therapeutic ultrasonic wave in a predetermined position in said scanning range; and
optical observation means (119 ) arranged on the side of the distal end of said insertion part (107), having an illumination window for outputting illumination light and an observation window for optically observing an object which is illuminated by said illumination light, said optical observation means (119) having an optical observation range which includes a direction in which said therapeutic ultrasonic wave is emitted,
wherein the focused therapeutic transducer (120), the observation ultrasonic transducer (118) and the optical observation means (119) are arranged in this order from the distal end of the insertion part (107).

2. An ultrasonic probe according to claim 1, further comprising a bendable part (115, at the side of the distal end of said insertion part.

3. An ultrasonic probe according to claim 1 or 2, wherein said insertion part (107) has a channel (142) therein into which a treatment tool is insertable.

4. An ultrasonic probe according to claim 1, 2 or 3, further comprising cooling means for cooling said therapeutic ultrasonic transducer (120 ).

5. An ultrasonic probe according to claim 4, wherein said cooling means has a line for supplying cooling liquid for cooling said therapeutic ultrasonic transducer, to a location in the vicinity of said therapeutic ultrasonic transducer (120).

6. An ultrasonic probe according to claim 1, further comprising a receiving coil for receiving a nuclear magnetic resonance signal for nuclear magnetic resonance imaging, said coil being placed on the distal end of said insertion part.

7. An ultrasonic probe according to claim 1, wherein said therapeutic ultrasonic transducer has a plurality of ultrasonic transducers which have a different distance to the focusing point.

8. An ultrasonic diagnosis and therapy system comprising:
ultrasonic observation and therapy means according to claims 1 to 7,
signal processing means (105A) for conducting signal processing which generates a signal driving said observation ultrasonic transducer (120, ) and which generates an image signal from an ultrasonic signal which is received by said observation ultrasonic transducer (118);
drive means (105B) for driving said therapeutic ultrasonic transducer (120 ) to generate a drive signal for generating a therapeutic ultrasonic wave;
display means (106) to which said image signal is inputted to thereby display said ultrasonic image corresponding to said image signal; and
scanning means for scanning said observation ultrasonic wave under a state in which a focusing point of said therapeutic ultrasonic wave is locked to a predetermined position within a scanning range of said observation ultrasonic wave.

9. A system according to claim 8, wherein said scanning means has mechanical scanning means (161b) for rotating said observation ultrasonic transducer (118,161a ), equal to or more than a predetermined angle which covers said scanning range, wherein said focusing point exists in an irradiation direction of said observation ultrasonic wave at the time said observation ultrasonic transducer (118 ) is rotated through a certain angle.

10. A system according to claim 8, wherein manipulation of transmission and receiving of said observation ultrasonic wave is possible in parallel with manipulation which irradiates said therapeutic ultrasonic wave to said focusing point.

11. A system according to claim 8, wherein said observation ultrasonic transducer (118 ) and said therapeutic ultrasonic transducer (120) are driven by respective frequencies which are different from each other.

12. A system according to claim 8, wherein said therapeutic ultrasonic transducer (120 ) has distance modification means for modifying a distance to the focusing point.

13. A system according to claim 8, wherein said display means (106) has head mount displays having respective display faces thereof in front of eyes of an operator.

## Patentansprüche

1. Ultraschallsonde (102A, 102B) mit:
einem langgestreckten Einführungsteil (107), der in ein Zölom einführbar ist;
einem fokussierten therapeutischen Ultraschallwandler (120), der entlang des distalen Endes des Einführungsteils angeordnet ist, zum Emittieren einer therapeutischen Ultraschallwelle zum Durchführen einer Therapie an einem Objekt;
einem Beobachtungsultraschallwandler (118) zum Erfassen eines Ultraschallbildes durch Senden und Empfangen einer Beobachtungsultraschallwelle über einen Abtastbereich, der eine Richtung umfasst, in der die therapeutische Ultraschallwelle emittiert wird, wobei der Beobachtungsultraschallwandler (118) entlang des distalen Endes des Einführungsteils (107) angeordnet ist;
einem Verriegelungsmittel zum Verriegeln eines Brennpunkts (123a) der therapeutischen Ultraschallwelle in einer vorbestimmten Position in dem Abtastbereich; und
einem optischen Beobachtungsmittel (119), das an der Seite des distalen Endes des Einführungsteils (107) angeordnet ist und ein Beleuchtungsfenster zum Aussenden von Beleuchtungslicht und ein Beobachtungsfenster zum optischen Beobachten eines Gegenstandes, der mit dem Beleuchtungslicht beleuchtet wird, aufweist, wobei das optische Beobachtungsmittel (119) einen optischen Beobachtungsbereich aufweist, der eine Richtung umfasst, in der die therapeutische Ultraschallwelle emittiert wird, wobei der fokussierte therapeutische Wandler (120), der Beobachtungsultraschallwandler (118) und das optische Beobachtungsmittel (119) in der Reihenfolge vom distalen Ende des Einführungsteils (107) angeordnet sind.

2. Ultraschallsonde nach Anspruch 1, welche ferner einen biegsamen Teil (115) an der Seite des distalen Endes des Einführungsteils umfasst.

3. Ultraschallsonde nach Anspruch 1 oder 2, wobei der Einführungsteil (107) einen Kanal (142) darin aufweist, in den ein Behandlungsinstrument einführbar ist.

4. Ultraschallsonde nach Anspruch 1, 2 oder 3, welche ferner ein Kühlmittel zum Kühlen des therapeutischen Ultraschallwandlers (120) umfasst.

5. Ultraschallsonde nach Anspruch 4, wobei das Kühlmittel eine Leitung zum Zuführen von Kühlflüssigkeit zum Kühlen des therapeutischen Ultraschallwandlers zu einem Ort in der Nähe des therapeutischen Ultraschallwandlers (120) aufweist.

6. Ultraschallsonde nach Anspruch 1, welche ferner eine Empfangsspule zum Empfangen eines kernmagnetischen Resonanzsignals für eine kernmagnetische Resonanzabbildung umfasst, wobei die Spule am distalen Ende des Einführungsteils angeordnet ist.

7. Ultraschallsonde nach Anspruch 1, wobei der therapeutische Ultraschallwandler eine Vielzahl von Ultraschallwandlern aufweist, die einen unterschiedlichen Abstand zum Brennpunkt aufweisen.

8. Ultraschalldiagnose- und Therapiesystem mit:
einem Ultraschallbeobachtungs- und Therapiemittel nach den Ansprüchen 1 bis 7,
einem Signalverarbeitungsmittel (105A) zum Durchführen einer Signalverarbeitung, die ein Signal erzeugt, das den Beobachtungsultraschallwandler (120) [(118)] ansteuert, und die ein Bildsignal aus einem Ultraschallsignal erzeugt, das von dem Beobachtungsultraschallwandler (118) empfangen wird,
einem Ansteuermittel (105B) zum Ansteuern des therapeutischen Ultraschallwandlers (120), um ein Ansteuersignal zum Erzeugen einer therapeutischen Ultraschallwelle zu erzeugen;
einem Anzeigemittel (106), in das das Bildsignal eingegeben wird, um dadurch das Ultraschallbild entsprechend dem Bildsignal anzuzeigen; und
einem Abtastmittel zum Abtasten der Beobachtungsultraschallwelle unter einem Zustand, in dem ein Brennpunkt der therapeutischen Ultraschallwelle in einer vorbestimmten Position innerhalb eines Abtastbereichs der Beobachtungsultraschallwelle verriegelt wird.

9. System nach Anspruch 8, wobei das Abtastmittel ein mechanisches Abtastmittel (161b) zum Drehen des Beobachtungsultraschallwandlers (118, 161a) um gleich oder mehr als einen vorbestimmten Winkel, der den Abtastbereich abdeckt, aufweist, wobei der Brennpunkt in einer Bestrahlungsrichtung der Beobachtungsultraschallwelle zu dem Zeitpunkt, zu dem der Beobachtungsultraschallwandler (118) über einen bestimmten Winkel gedreht wird, existiert.

10. System nach Anspruch 8, wobei eine Handhabung des Sendens und des Empfangs der Beobachtungsultraschallwelle parallel mit der Handhabung möglich ist, die die therapeutische Ultraschallwelle auf den Brennpunkt ausstrahlt.

11. System nach Anspruch 8, wobei der Beobachtungsultraschallwandler (118) und der therapeutische Ultraschallwandler (120) durch jeweilige Frequenzen angesteuert werden, die voneinander verschieden sind.

12. System nach Anspruch 8, wobei der therapeutische Ultraschallwandler (120) ein Abstandsmodifikationsmittel zum Modifizieren eines Abstands zum Brennpunkt aufweist.

13. System nach Anspruch 8, wobei das Anzeigemittel (106) am Kopf befestigte Anzeigen aufweist, deren jeweilige Anzeigeflächen sich vor den Augen einer Bedienperson befinden.

## Revendications

1. Sonde à ultrasons (102A, 102B) comprenant :
une partie d'insertion allongée (107) pouvant être insérée dans le coelome,
un transducteur à ultrasons thérapeutique focalisé (120) placé le long de l'extrémité distale de ladite partie d'insertion, destiné à émettre une onde ultrasonore thérapeutique en vue d'effectuer une thérapie sur un sujet,
un transducteur à ultrasons d'observation (118) destiné à acquérir une image par ultrasons en émettant et en recevant une onde ultrasonore d'observation sur une plage de balayage qui comprend une direction dans laquelle ladite onde ultrasonore thérapeutique est émise, ledit transducteur à ultrasons d'observation (118) étant placé le long de l'extrémité distale de ladite partie d'insertion (107),
un moyen de blocage destiné à bloquer un point de focalisation (123a) de ladite onde ultrasonore thérapeutique à une position prédéterminée dans ladite plage de balayage, et
un moyen d'observation optique (119) agencé du côté de l'extrémité distale de ladite partie d'insertion (107), comportant une fenêtre d'illumination destinée à fournir en sortie une lumière d'illumination et une fenêtre d'observation destinée à observer optiquement un objet qui est illuminé par ladite lumière d'illumination, ledit moyen d'observation optique (119) comportant une plage d'observation optique qui comprend une direction dans laquelle ladite onde ultrasonore thérapeutique est émise, dans laquelle le transistor thérapeutique focalisé (120), le transducteur à ultrasons d'observation (118) et le moyen d'observation optique (119) sont agencés dans cet ordre depuis l'extrémité distale de la partie d'insertion (107).

2. Sonde à ultrasons selon la revendication 1, comprenant en outre une partie pouvant être pliée (115) au niveau du côté de l'extrémité distale de ladite partie d'insertion.

3. Sonde à ultrasons selon la revendication 1 ou 2, dans laquelle ladite partie d'insertion (107) comporte un canal (142) dans celle-ci, dans lequel un outil de traitement peut être inséré.

4. Sonde à ultrasons selon la revendication 1, 2 ou 3, comprenant en outre un moyen de refroidissement destiné à refroidir ledit transducteur à ultrasons thérapeutique (120).

5. Sonde à ultrasons selon la revendication 4, dans lequel ledit moyen de refroidissement comporte une conduite destinée à fournir un liquide de refroidissement, en vue de refroidir ledit transducteur à ultrasons thérapeutique, vers un emplacement à proximité dudit transducteur à ultrasons thérapeutique (120).

6. Sonde à ultrasons selon la revendication 1, comprenant en outre une bobine de réception destinée à recevoir un signal de résonance magnétique nucléaire en vue de former une image par résonance magnétique nucléaire, ladite bobine étant placée sur l'extrémité distale de ladite partie d'insertion.

7. Sonde à ultrasons selon la revendication 1, dans laquelle ledit transistor à ultrasons thérapeutique comporte une pluralité de transducteurs à ultrasons qui présentent une distance différente par rapport au point de focalisation.

8. Système de diagnostic et de thérapie par ultrasons comprenant :
un moyen d'observation et de thérapie par ultrasons selon les revendications 1 à 7,
un moyen de traitement du signal (105A) destiné à réaliser un traitement du signal qui génère un signal attaquant ledit transducteur à ultrasons d'observation (120) et qui génère un signal d'image à partir d'un signal ultrasonore qui est reçu par ledit transducteur à ultrasons d'observation (118),
un moyen d'attaque (105B) destiné à attaquer ledit transducteur à ultrasons thérapeutique (120) afin de générer un signal d'attaque en vue de générer une onde ultrasonore thérapeutique,
un moyen d'affichage (106) auquel ledit signal d'image est appliqué en entrée afin d'afficher ainsi ladite image par ultrasons correspondant audit signal d'image, et
un moyen de balayage destiné à balayer ladite onde ultrasonore d'observation dans un état où un point de focalisation de ladite onde ultrasonore thérapeutique est bloqué à une position prédéterminée à l'intérieur d'une plage de balayage de ladite onde ultrasonore d'observation.

9. Système selon la revendication 8, dans lequel ledit moyen de balayage comporte un moyen de balayage mécanique (161b) destiné à entraîner en rotation ledit transducteur à ultrasons d'observation (118, 161a) d'un angle supérieur ou égal à un angle prédéterminé qui couvre ladite plage de balayage, où ledit point de focalisation existe dans une direction de rayonnement de ladite onde ultrasonore d'observation au moment où ledit transducteur à ultrasons d'observation (118) est entraîné en rotation d'un certain angle.

10. Système selon la revendication 8, dans lequel la manipulation d'émission et de réception de ladite onde ultrasonore d'observation est possible parallèlement à la manipulation qui fait rayonner ladite onde ultrasonore thérapeutique sur ledit point de focalisation.

11. Système selon la revendication 8, dans lequel ledit transducteur à ultrasons d'observation (118) et ledit transducteur à ultrasons thérapeutique (120) sont attaqués par des fréquences respectives qui sont différentes l'une de l'autre.

12. Système selon la revendication 8, dans lequel ledit transistor (120) à ultrasons thérapeutique comporte un moyen de modification de distance destiné à modifier une distance par rapport au point de focalisation.

13. Système selon la revendication 8, dans lequel ledit moyen d'affichage (106) comporte des dispositifs d'affichage de montures de tête comportant des faces d'affichage respectives de ceux-ci devant les yeux d'un opérateur.
